(19) Europäisches Patentamt
European Patent Office
Office européen des brevets

(11) EP 0 671 164 B1

(12) **FASCICULE DE BREVET EUROPEEN**

(45) Date de publication et mention de la délivrance du brevet:
**10.06.1998 Bulletin 1998/24**

(51) Int. Cl.$^6$: **A61K 7/48**, A61K 7/42

(21) Numéro de dépôt: **95400269.7**

(22) Date de dépôt: **09.02.1995**

(54) **Compositon pour lutter contre les taches et/ou vieillissement de la peau, ses utilisations**

Zusammensetzung zur Bekämpfung von Hautflecken und/oder Hautalterung und ihre Verwendungen

Skin whitening and/or anti aging composition and uses thereof

(84) Etats contractants désignés:
**AT BE CH DE ES FR GB IT LI NL SE**

(30) Priorité: **08.03.1994 FR 9402656**

(43) Date de publication de la demande:
**13.09.1995 Bulletin 1995/37**

(73) Titulaire: **L'OREAL**
**75008 Paris (FR)**

(72) Inventeur: **Cabanne, Françoise**
**F-92320 Châtillon (FR)**

(74) Mandataire: **Dodin, Catherine**
**L'Oreal-D.P.I.,**
**90, rue du Général Roguet**
**92583 Clichy Cédex (FR)**

(56) Documents cités:
**EP-A- 0 381 057** **EP-A- 0 531 192**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 671 164 B1

## Description

L'invention se rapporte à une composition cosmétique et/ou dermatologique destinée à prévenir et/ou lutter contre les taches cutanées et/ou contre le vieillissement de la peau. Cette composition se présente sous forme d'une crème blanche onctueuse pouvant être appliquée sur le visage, le corps et/ou les jambes d'êtres humains et plus spécialement sur les mains.

L'invention se rapporte aussi à une utilisation de cette composition pour le traitement cosmétique de la peau, à l'utilisation de cette composition pour la préparation d'une crème destinée au traitement dermatologique de la peau et à un procédé de traitement cosmétique consistant à appliquer cette composition sur la peau.

Au cours du processus de vieillissement, il apparaît différents signes sur la peau, très caractéristiques de ce vieillissement, se traduisant notamment par une modification de la structure et des fonctions cutanées. Ce vieillissement est de nature physiologique mais il peut être également photoinduit, c'est-à-dire dû à une exposition répétée de la peau à la lumière solaire, notamment ultraviolette. L'action de cette lumière sur les constituants de la peau et sur le sébum sécrété par la peau entraîne en particulier la formation de radicaux libres oxygénés. Or, ces radicaux provoquent des dégats importants, notamment dans les membranes cellulaires (perméabilité des membranes), les noyaux des cellules (mutation par action sur l'ARN ou ADN), et les tissus (nécroses, dégénérescence) ; il est donc nécessaire de protéger la peau contre ces radicaux libres.

Les principaux signes cliniques du vieillissement cutané sont notamment l'apparition de ridules et de rides profondes, en augmentation avec l'âge. On constate en particulier une désorganisation du "grain" de la peau, c'est-à-dire que le micro-relief est moins régulier et présente un caractère anisotrope.

Par ailleurs, le teint de la peau est généralement modifié ; il apparaît plus pâle et plus jaune ceci semble être dû essentiellement à une désorganisation de la microcirculation (moins d'hémoglobine au niveau du derme papillaire). De plus, de nombreuses taches colorées et/ou plus foncées apparaissent à la surface de la peau, et plus spécialement sur les mains, conférant à la peau une hétérogénéité. En général, ces taches sont dues à une production importante de mélanine dans l'épiderme et/ou le derme de la peau. Dans certains cas, ces taches peuvent devenir cancéreuses. Aussi, on cherche de plus en plus à diminuer, voire éliminer, ces taches. Par ailleurs, il peut exister sur certaines zones de la peau des irritations diffuses et parfois des télangiectasies.

Un autre signe clinique du vieillissement est l'aspect sec et rêche de la peau qui est dû essentiellement à une desquamation plus importante ; ces squames, en diffractant les rayons lumineux, participent aussi à l'aspect un peu gris du teint.

On constate enfin une perte de fermeté et de tonicité de la peau qui, comme pour les rides et les ridules, s'explique du moins en partie par une atrophie dermique et épidermique ainsi qu'un applatissement de la formation dermoépidermique ; la peau est moins épaisse et plus flasque, et l'épaisseur de l'épiderme diminue.

On constate donc que les signes cliniques du vieillissement cutané résultent essentiellement d'un dysfonctionnement des principaux mécanismes biologiques intervenant au niveau de la peau.

Aussi, la composition selon l'invention est une composition apte à prévenir et/ou lutter contre l'apparition du vieillissement et les signes de vieillissement existants, comme les rides, les ridules, à prévenir et/ou lutter contre les taches de pigmentation de la peau, quelle que soit leur origine, ainsi qu'à protéger la peau notamment par suppression de la formation de radicaux libres oxygénés.

Il est connu, par le document J02-200622, d'associer l'acide kojique à un composé choisi parmi les dérivés de l'acide para-aminobenzoïque, salicylique ou méthoxy-cinnamique et les dérivés de la benzophénone pour prévenir les érythèmes et la pigmentation de la peau, induits par les rayons ultaviolets.

Par ailleurs, il est connu du document FR-A-2 680 466 une composition blanchissante contenant un flavonoïde, de l'acide kojique, du méthoxydibenzoylméthane de butyle et du méthoxycinnamate, servant de filtres ultraviolets. Le méthoxydibenzoylméthane de butyle présente l'inconvénient d'être photoinstable (c'est-à-dire déstabilisé en présence de lumière, ce qui est particulièrement gênant pour un filtre). Aussi, pour améliorer sa stabilité, il est nécessaire de lui associer un autre filtre du type cinnamate, compliquant la fabrication de la composition.

En outre, il existe sur le marché de nombreuses compositions destinées à lutter contre les signes du vieillissement.

Malheureusement, l'efficacité de ces compositions connues est souvent encore insuffisante.

L'invention a justement pour objet une composition plus efficace que celle de l'état de la technique pour prévenir et/ou lutter contre le vieillissement de la peau et/ou les taches cutanées.

Selon une caractéristique essentielle de l'invention, cette composition contient dans un milieu cosmétiquement et/ou dermatologiquement acceptable de l'acide kojique et un filtre ultraviolet choisi dans le groupe constitué du benzylidène camphre et de ses dérivés.

Ainsi, de façon surprenante, la demanderesse a découvert que l'utilisation simultanée d'acide kojique et de benzylidène camphre et/ou l'un de ses dérivés permettait d'atténuer les rides et les ridules, de modifier le teint de la peau qui apparaît plus rosé, d'effacer les taches de pigmentation, de supprimer les squames et de donner une consistance plus élastique à la peau.

En particulier, la composition de l'invention permet d'atténuer ou estomper les taches pour 50 % des femmes ayant appliqué cette composition sur les mains. Il s'agit en fait d'un effet curatif inattendu, jamais atteint avec les produits à l'acide kojique ou à tout autre actif de dépigmentation, actuellement disponibles sur le marché.

Etant donné l'efficacité relativement faible de l'acide kojique, il est tout-à-fait surprenant que le benzylidène camphre et/ou ses dérivés augmentent, de façon considérable, son efficacité anti-tache.

Les dérivés de benzylidène camphre utilisables dans l'invention sont, en particulier, les dérivés sulfonés et/ou sulfonatés.

En particulier, les dérivés de benzylidène camphre utilisables dans l'invention présente la formule générale (a) suivante :

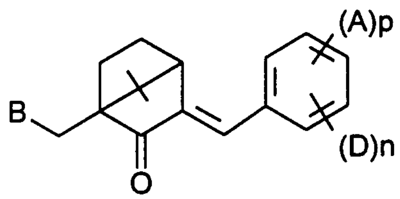

(a)

dans laquelle :

B représente -H ou $-SO_3H$,

$0 \leq p \leq 1$ avec B = $-SO_3H$ quand p = 0,

$0 \leq n \leq 4$,

D représente un ou plusieurs radicaux alkyle ou alcoxy, identiques ou différents quand $n \geq 2$, linéaires ou ramifiés contenant de 1 à 18 atomes de carbone environ, un radical halogéno, un radical hydroxyle.

A, de préférence en méta ou en para, représente
soit un radical $SO_3H$ ;
soit un groupement :

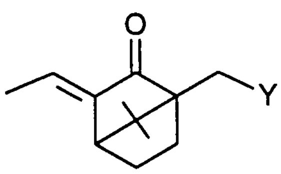

dans lequel Y représente H ou $SO_3H$ ;
soit un groupement:

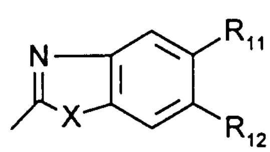

dans lequel :

$R_{11}$ désigne un atome d'hydrogène, un radical alkyle ou alcoxy, linéaire ou ramifié contenant de 1 à 6 atomes de carbone environ ou le radical $-SO_3H$, $R_{11}$ étant $-SO_3H$ lorsque B = -H,

$R_{12}$ désigne un atome d'hydrogène, un radical alkyle ou alcoxy, linéaire ou ramifié, contenant de 1 à 6 atomes de carbone environ,

X est un atome d'oxygène, de soufre ou un groupement -NR-, R étant un atome d'hydrogène ou un radical alkyle, linéaire ou ramifié, contenant de 1 à 6 atomes de carbone environ,

et dans laquelle au moins une fonction $-SO_3H$ est éventuellement neutralisée.

La neutralisation d'une ou plusieurs fonctions peut être obtenue à l'aide d'une base généralement utilisée dans le domaine cosmétique comme la soude, la triéthanolamine, la potasse.

On peut citer comme exemples particuliers de composés de formule (a) les dérivés de formules (I), (II), (III) suivantes :

*Formule (I) :*

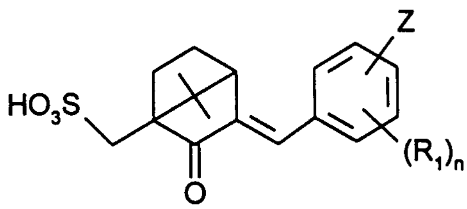

(I)

dans laquelle :

- Z, de préférence en position para ou méta, désigne un groupement

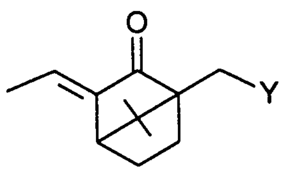

dans lequel Y représente -H ou $-SO_3H$, éventuellement neutralisé,

- n est égal à 0 ou est un nombre allant de 1 à 4 ($0 \leq n \leq 4$),

- $R_1$, représente un ou plusieurs radicaux alkyle ou alkoxy, identiques ou différents, linéaires ou ramifiés, contenant de 1 à 4 atomes de carbone environ.

Un composé de formule (I) particulièrement préféré est celui correspondant à n = 0 , Z en position para et Y = $-SO_3H$ : l'acide benzène 1,4 [di(3-méthylidène-campho 10-sulfonique)], appelé aussi (selon la nomenclature CTFA - 5ème édition) l'acide téréphtalylidène-di-camphosulfonique.

*Formule (II) :*

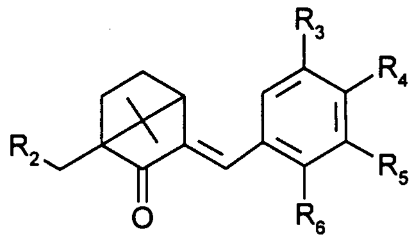

(II)

dans laquelle :

- $R_2$ désigne un atome d'hydrogène ou un radical $-SO_3H$,
- $R_3$, $R_4$, $R_5$ et $R_6$, identiques ou différents, représentent un groupement hydroxyle, un radical alkyle ayant de 1 à 4 atomes de carbone environ, linéaire ou ramifié, un radical alcényle ayant de 2 à 4 atomes de carbone environ, linéaire ou ramifié, un radical alcoxy ayant de 1 à 4 atomes de carbone, linéaire ou ramifié, un radical alcényloxy ayant de 2 à 4 atomes de carbone, linéaire ou ramifié, un radical halogéno ; de plus un radical $R_3$ à $R_6$ seulement peut être un radical $-SO_3H$, au moins un des radicaux $R_3$ à $R_6$ désignant le radical $-SO_3H$ quand $R_2$ est un atome d'hydrogène. Une ou plusieurs fonctions $-SO_3H$ peuvent aussi être neutralisées.

On peut citer comme exemples particuliers les composés suivants de formule (II) dans laquelle :

- $R_4$ désigne le radical $-SO_3H$ en position para du benzylidènecamphre et $R_2$, $R_3$, $R_5$ et $R_6$ désignent chacun un atome d'hydrogène, c'est-à-dire l'acide 4'-sulfo 3-benzylidènecamphre.
- $R_3$, $R_4$, $R_5$ et $R_6$ désignent chacun un atome d'hydrogène et $R_2$ désigne un radical $-SO_3H$, c'est-à-dire l'acide 3-benzylidène campho-10 sulfonique.
- $R_4$ désigne un radical méthyle en position para du benzylidènecamphre, $R_5$ un radical $-SO_3H$ et $R_2$, $R_3$ et $R_6$ représentent un atome d'hydrogène, c'est-à-dire l'acide 4'-méthyl 3'-sulfo 3-benzylidènecamphre.
- $R_4$ désigne un atome de chlore en position para du benzylidènecamphre, $R_5$ un radical $-SO_3H$ et $R_2$, $R_3$ et $R_6$ représentent un atome d'hydrogène, c'est-à-dire l'acide 4'-chloro 3'-sulfo 3-benzylidènecamphre.
- $R_4$ désigne un radical méthyle en position para du benzylidènecamphre, $R_3$, $R_5$ et $R_6$ désignent un atome d'hydrogène et $R_2$ désigne un radical $-SO_3H$, c'est-à-dire l'acide 4'-méthyl 3-benzylidène campho 10-sulfonique.
- $R_2$ représente un radical $-SO_3H$, $R_3$ est un radical méthyle, $R_4$ un atome d'hydrogène, $R_5$ un radical tertiobutyle, $R_6$ un radical hydroxyle, c'est-à-dire l'acide (3-t-butyl 2-hydroxy 5-méthyl) 3-benzylidène campho-10-sulfonique.
- $R_2$ représente un radical $-SO_3H$, $R_3$ est un radical méthoxy, $R_4$ un atome d'hydrogène, $R_5$ un radical tertiobutyle, $R_6$ un radical hydroxyle, c'est-à-dire l'acide (3-t-butyl 2-hydroxy 5-méthoxy) 3-benzylidène campho-10-sulfonique.
- $R_2$ représente un radical $-SO_3H$, $R_3$ et $R_5$ désignent chacun un radical tertiobutyle, $R_4$ un radical hydroxyle, $R_6$ un atome d'hydrogène, c'est-à-dire l'acide (3,5-diterbutyl 4-hydroxy) 3-benzylidène campho-10-sulfonique.
- $R_4$ représente un radical méthoxy en para, $R_5$ représente $-SO_3H$, les radicaux $R_2$, $R_3$ et $R_6$ représentent H, c'est-à-dire l'acide 4'-méthoxy 3'-sulfo-3-benzylidène camphre.
- $R_2$ désigne un radical $-SO_3H$, $R_3$ et $R_6$ représentent H, $R_4$ et $R_5$ formant un radical méthylènedioxy, c'est-à-dire l'acide 3-(4,5-méthylènedioxy) benzylidène campho-10-sulfonique.

- $R_2$ représente un radical $-SO_3H$, $R_4$ un radical méthoxy et les radicaux $R_3$, $R_5$ et $R_6$ représentent H, c'est-à-dire l'acide 3-(4-méthoxy) benzylidène campho-10-sulfonique.
- $R_2$ représente un radical $-SO_3H$, $R_4$ et $R_5$ sont tous deux un radical méthoxy et les radicaux $R_3$ et $R_6$ représentent H, c'est-à-dire l'acide 3-(4,5-diméthoxy) benzylidène campho-10-sulfonique.
- $R_2$ représente un radical $-SO_3H$, $R_4$ est un radical n-butoxy et les radicaux $R_3$, $R_5$ et $R_6$ représentent un atome d'hydrogène, c'est-à-dire l'acide 3-(4-n.butoxy) benzylidène campho-10-sulfonique.
- $R_2$ représente un radical $-SO_3H$, $R_4$ est un radical n-butoxy, $R_5$ est un radical méthoxy et $R_3$ et $R_6$ désignent tous deux un atome d'hydrogène, c'est-à-dire l'acide 3-(4-n.butoxy 5-méthoxy) benzylidène campho-10-sulfonique.

*Formule (III) :*

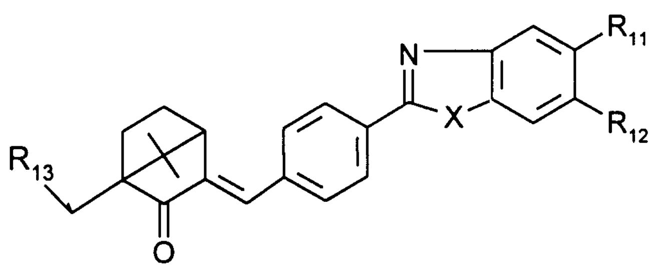

(III)

dans laquelle :

- $R_{11}$ désigne un atome d'hydrogène, un radical alkyle ou alcoxy, linéaire ou ramifié, contenant de 1 à 6 atomes de carbone environ ou un radical - $SO_3H$,
- $R_{12}$ désigne un atome d'hydrogène, un radical alkyle ou alcoxy, linéaire ou ramifié, contenant de 1 à 6 atomes de carbone environ,
- $R_{13}$ désigne un atome d'hydrogène ou un radical $-SO_3H$,
- l'un au moins des radicaux $R_{11}$ et $R_{13}$ désignant un radical $-SO_3H$,
- X est un atome d'oxygène ou de soufre ou un groupement -NR-, R étant un atome d'hydrogène ou un radical alkyle, linéaire ou ramifié, contenant de 1 à 6 atomes de carbone environ.

On peut citer comme exemple particulier de composé de formule (III) : le composé dans lequel X désigne un radical -NH-, $R_{11}$ désigne un radical $-SO_3H$, $R_{12}$ et $R_{13}$ désignent tous deux un atome d'hydrogène, c'est-à-dire l'acide 2-[4-(camphométhylidène)phényl]benzimidazole-5-sulfonique.

Les composés de structures (I), (II), (III) sont décrits dans le brevet US 4.585.597 et les brevets FR 2.236.515, 2.282.426, 2.645.148, 2.430.938, 2.592.380.

On peut citer comme autres exemples de dérivés du benzylidène camphre utilisables dans l'invention les composés de formule générale (b) suivante :

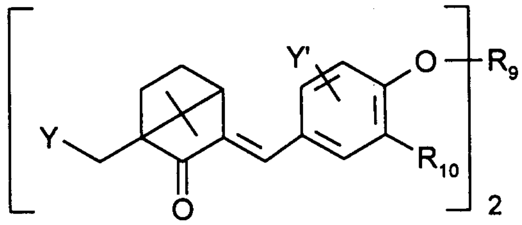

(b)

dans laquelle :

- $R_9$ désigne un radical divalent : $-(CH_2)_m-$ ou $-CH_2$ $-CHOH-CH_2-$, m étant un nombre entier allant de 1 à 10 ($1 \leq m \leq 10$),
- $R_{10}$ désigne un atome d'hydrogène, un radical alcoxy contenant de 1 à 4 atomes de carbone environ ou un radical divalent - O - relié au radical $R_9$ lorsque celui-ci est divalent lui aussi,
- Y et Y' désignent un atome d'hydrogène ou un radical $-SO_3H$, au moins un de ces radicaux Y ou Y' est différent de l'hydrogène. Là encore la fonction - $SO_3H$ peut être neutralisée.

On peut citer comme exemples particuliers les composés suivants de formule (b) dans laquelle Y représente - $SO_3H$, Y' est -H, $R_{10}$ est H et $R_9$ est $-CH_2-CH_2-$, c'est à dire l'acide éthylène bis [(4'-oxy benzylidène) 3-campho-10 sulfonique].

Selon l'invention, la quantité d'acide kojique est celle classiquement utilisée dans le domaine cosmétique ou dermatologique. A titre d'exemple, on peut utiliser de 0,05 % à 10 % en poids par rapport au poids total de la composition et de préférence de 0,5 % à 2 %.

De même, la quantité de dérivé de benzylidène camphre utilisable dans l'invention est celle généralement utilisée dans les domaines concernés. En pratique, on utilise de 0,1 % à 10 % en poids par rapport au poids total de la composition et de préférence de 0,1 % à 5 %.

La composition de l'invention peut présenter toutes les formes galéniques normalement utilisées pour une application topique telles que les solutions, les gels aqueux ou hydroalcooliques, les émulsions huile-dans-eau ou eau-dans-huile, et plus particulièrement les gouttelettes d'huile dispersées par des sphérules dans une phase aqueuse. Ces sphérules peuvent être des nanoparticules polymériques telles que les nanosphères et les nanocapsules ou mieux des vésicules lipidiques. La composition de l'invention peut se présenter sous forme d'une crème, d'une pommade, d'une lotion ou d'un sérum.

Les huiles utilisables dans l'invention sont celles généralement utilisées dans les domaines concernés. Elles peuvent être végétales, minérales ou synthétiques, éventuellement siliconées et/ou fluorées.

L'invention peut également contenir des adjuvants hydrophiles ou lipophiles comme des gélifiants, des conservateurs, des opacifiants, des émulsionnants, des coémulsionnants, des neutralisants, des parfums et leurs solubilisants ou peptisants, des colorants, des pigments, des charges, ainsi que des actifs lipophiles ou hydropliles autres que l'acide kojique, le benzylidène camphre et l'un de ses dérivés.

Les quantités d'huile et d'eau sont généralement celles utilisées dans les domaines considérés et sont fonction de la forme galénique de la composition.

Pour une émulsion huile-dans-eau ou une dispersion d'huile dans de l'eau par des sphérules lipidiques, l'huile peut représenter de 2 % à 40 % en poids par rapport au poids total de la composition.

De même les adjuvants sont utilisés en quantité habituelle et peuvent représenter, au total, de 0,1 % à 20 % en poids. Leur quantité est fonction de leur nature.

Bien que la composition de l'invention puisse être appliquée par voie topique sur toutes les parties du corps et du visage, elle est particulièrement intéressante pour le traitement des mains. Aussi, l'invention se rapporte encore à une utilisation de la composition ci-dessus pour un traitement cosmétique des mains.

L'invention a aussi pour objet une utilisation de la composition définie ci-dessus pour le traitement cosmétique des rides et/ou les ridules de la peau ainsi qu'une utilisation de cette composition pour tonifier, hydrater et/ou raffermir la peau.

L'invention a encore pour objet une utilisation de la composition définie ci-dessus pour le traitement cosmétique des taches cutanées dues au vieillissement, présentes sur le visage et/ou le corps, y compris les mains et le cuir chevelu ainsi que pour la préparation d'une crème destinée au traitement des taches de la peau d'origine pathologique.

L'invention a encore pour objet un procédé de traitement cosmétique de la peau, consistant à appliquer la composition définie ci-dessus sur la peau.

Les tests suivants ont mis en évidence les avantages de la présente invention. Le but des tests a été de montrer l'aptitude des compositions de l'invention à s'opposer à la pigmentation et à la formation d'érythème de la peau soumise aux UV.

Les tests ont été effectués sur 9 sujets avec 3 compositions, la première selon l'invention contenant l'association d'acide kojique et d'acide téréphtalylidène di-camphosulfonique, la seconde illustrant l'état de la technique, contenant l'association d'acide kojique et d'oxybenzophénone, et une troisième contenant le milieu exempt d'acide kojique et de filtres (placebo). Les sujets ont appliqué les compositions 2 fois par jour matin et soir durant 24 jours et, les jours d'irradiation, ils ont recu une application supplémentaire 2 h avant l'irradiation.

L'irradiation a été réalisée à l'aide d'une lampe au xénon 2500 W Osram munie d'un filtre WG 320 Schott de 1 mm d'épaisseur coupant une très grande partie des UVB ainsi qu'un filtre à eau coupant les IR. Ce système reproduit un spectre solaire enrichi en UVA.

L'irradiation a été réalisée sur 9 jours. On a tout d'abord déterminé la MED (dose érythémateuse minimale) des sujets (selon la recommandation de la FDA du 25.04. 1978) ; le second jour, l'irradiation a été de 0,75 MED, le troisième de 1 MED ; le quatrième et le cinquième jour, il n'y a pas eu d'irradiation ; le sixième jour, l'irradiation a été de 1,25 MED ; puis elle a été de 1,5 MED pendant trois jours consécutifs. Il n'y a pas eu d'irradiation après le neuvième jour, mais on a continué d'appliquer les compositions et de faire les mesures jusqu'au 24 ème jour.

On a déterminé d'une part le pourcentage d'inhibition de l'érythème grâce aux première et seconde compositions par rapport au placebo par une évaluation visuelle classiquement utilisée pour la détermination des SPF (facteur de protection solaire), d'autre part le pourcentage d'inhibition de la pigmentation grâce à ces première et seconde compositions par rapport au placebo par une mesure de l'assombrissement de la peau à l'aide d'un chromamètre Minolta Lab. Les pourcentages d'inhibition sont calculés par rapport au placebo selon les formules suivantes :

$$\frac{\text{Inhibition de composition (1) ou (2) - inhibition du placebo}}{\text{Inhibition du placebo}} \times 100$$

| Associations | Inhibition de l'érythème | | Inhibition de la pigmentation | |
|---|---|---|---|---|
| | J3 | J9 | J9 | J24 |
| Acide kojique + acide téréphtalylidène di-camphosulfonique | + 54 % | + 78 % | + 48 % | + 82 % |
| Acide kojique + oxybenzophénone | + 36 % | + 59 % | + 35 % | + 62 % |

Ces tests montrent que l'acide téréphtalylidène di-camphosulfonique potentialise l'effet de l'acide kojique de manière nettement plus importante que l'oxybenzophénone ; l'inhibition de l'érythème et celle de la pigmentation avec la composition de l'invention sont très supérieures (20 % en plus) à celles obtenues avec la composition de l'art antérieur.

D'autres caractéristiques et avantages de l'invention ressortiront mieux de la description qui suit, donnée à titre illustratif et non limitatif. Dans les exemples ci-après de compositions cosmétiques et/ou dermatologiques conformes à l'invention, les compositions sont données en % pondéral.

**EXEMPLE 1** : Crème huile-dans-eau anti-tache

| Composition | | |
|---|---|---|
| *$A_1$* | Tri-stéarate de Sorbitane (émulsionnant) | 0,7 % |
| | Stéarate de polyéthylène glycol (40.OE) (émulsionnant) | 1,6 % |
| | Alcool cétylique (co-émulsionnant) | 3,2 % |
| | Mono,di,tri-palmitostéarate de glycéryle (émulsionnant) | 2,4 % |
| | Myristate de myristyle (huile) | 2 % |
| | Fraction liquide de beurre de karité (huile) | 2 % |
| | Para-hydroxybenzoate de propyle (conservateur) | 0,2 % |
| *$A_2$* | Cyclopentadiméthylsiloxane (huile) | 15 % |
| *B* | Eau déminéralisée qsp | 100 % |
| | Glycérol (hydratant) | 3 % |
| | Acide kojique | 1 % |
| | Para-hydroxybenzoate de méthyle (conservateur) | 0,2 % |
| *C* | Acide téréphtalylidène di-camphosulfonique dans l'eau à 33 % | 2 % |
| | Triéthanolamine (neutralisant) | 0,3 % |
| *D* | Parfum | 0,3 % |

**<u>Préparation de la phase $A_1$ + $A_2$</u>**

Les constituants de $A_1$ sont solubilisés à 80°C. Lorsque le mélange est limpide, la température est abaissée à 65°C et on ajoute $A_2$. Le mélange doit être limpide et homogène. On maintient la température de 65°C.

**<u>Fabrication</u>**

Dans un bécher de fabrication, les constituants de B sont solubilisés à 85 °C-90 °C. Après vérification de la limpidité, la température est ramenée à 65°C. On réalise l'émulsion, sous agitation, en versant ($A_1$+$A_2$) dans B. On poursuit le refroidissement sous agitation. A 40 °C on ajoute la phase C, puis on maintient l'agitation. Enfin, on ajoute le parfum et on laisse refroidir à 20 °C sous agitation.

**EXEMPLE 2** : Crème huile dans eau

## Composition

| | | | |
|---|---|---|---|
| $A_1$ | - Eau déminéralisée | | 10 % |
| | - Cholestérol | } | 1,5 % |
| | - Monostéarate de polyéthylène glycol ... | } (vésicules) | 1,5 % |
| | - Sel monosodique de n-(acide stéarique) d'-α-acide glutamique | } | 0,2 % |
| $A_2$ | - Eau déminéralisée | | 13 % |
| | - Glycérine (hydratant) | | 3 % |
| | - Phénoxyéthanol (conservateur) | | 0,7 % |
| B | - Huile d'amandes d'abricot | | 9 % |
| | - Huile de soja raffinée | | 4 % |
| | - Cyclopenta-diméthylsiloxane (huile) | | 10 % |
| | - Parahydroxy-benzoate de propyle | | 0,1 % |
| | - Parfum | | 0,3 % |
| C | - Polymère carboxyvinylique synthétisé dans le chlorure de méthylène (gélifiant) | | 0,7 % |
| | - Eau déminéralisée | | 36,45 % |
| | - Triéthanolamine (neutralisant) | | 0,7 % |
| D | - Eau déminéralisée | | 5 % |
| | - Acide kojique | | 1 % |
| E | - Acide téréphtalylidène di-camphosulfonique dans l'eau à 33 % | | 2,3 % |
| | - Triéthanolamine | | 0,6 % |

**Fabrication**

On fait fondre les constituants de $A_1$ à 100 °C. On laisse gonfler sous agitation pendant 1H30. Lorsque le mélange est homogène on ajoute $A_2$ ; on stabilise la température à 80 °C. On effectue alors deux passages à l'homogénéisateur haute pression pour former les vésicules.

On prépare B à 70 °C ; le mélange doit être limpide. On refroidit à 50 °C. On ajoute B dans A à 50 °C.

On fait alors deux passages à l'homogénéisateur haute pression pour disperser la phase grasse B. On refroidit à 30 °C.

On ajoute C (le gel aura été préalablement préparé dans l'eau à 80 °C, en saupoudrant le polymère carboxyvinylique ; après gonflement de ce dernier, on neutralise à la triéthanolamine sous agitation ; le gel doit être bien lisse). On ajoute D puis E. On maintient l'agitation pendant 5 minutes. La fabrication est terminée.

**EXEMPLE 3** : Gel

| Composition | | |
|---|---|---|
| *A* | Eau déminéralisée | 32 % |
| | Polymère carboxyvinylique | 0,45 % |
| | Triéthanolamine | 0,45 % |
| *B* | Eau déminéralisée | 59,8 % |
| | Glycérine | 3 % |
| | Para-hydroxybenzoate de méthyle | 0,2 % |
| | Acide kojique | 1 % |
| | Gomme de Xanthane (gélifiant) | 0,2 % |
| *C* | Acide téréphtalylidène di-camphosulfonique dans l'eau à 33 % | 2,3 % |
| | Triéthanolamine | 0,6 % |

__Fabrication__

Dans l'eau à 80 °C, on prépare le gel (polymère carboxyvinylique) comme dans l'exemple 2. Après solubilisation des constituants de B à 80 °C, on ajoute B dans A. On lisse et on laisse refroidir sous agitation à pales lentes. A 35 °C, on ajoute C. On laisse refroidir à 25 °C. La fabrication du gel est terminée.

**EXEMPLE 4 :** Lotion

| Composition | | |
|---|---|---|
| *A* | Triglycérides ricinoléiques hydrogénés oxyéthylénés (60 OE) (peptisant) | 0,09 % |
| | Parfum | 0,03 % |
| *B* | Eau déminéralisée qsp | 100 % |
| | Glycérine | 5,5 % |
| | Acide kojique | 1 % |
| | Acide citrique | 1 % |
| | Triéthanolamine à 99 % | 1,9 % |
| | Imidazolidinyl urée (conservateur) | 0,3 % |
| *C* | Acide téréphtalylidène di-camphosulfonique dans l'eau à 33 % | 2,3 % |
| | Triéthanolamine | 0,6 % |

__Fabrication__

On mélange les constituants de A à 40 °C. Lorsqu'ils sont parfaitement solubilisés, on ajoute successivement les constituants de B à température ambiante. On maintient l'agitation et on vérifie la bonne solubilisation des constituants. On ajoute C ; le mélange doit être limpide. La fabrication est terminée.

## Revendications

1. Composition pour prévenir et/ou lutter contre les taches cutanées et/ou contre le vieillissement de la peau, caractérisée en ce qu'elle contient dans un milieu cosmétiquement et/ou dermatologiquement acceptable de l'acide kojique et un filtre ultraviolet choisi dans le groupe constitué du benzylidène camphre et de ses dérivés.

2. Composition selon la revendication 1, caractérisée en ce que le filtre est un dérivé sulfoné ou sulfonaté du benzylidène camphre.

3. Composition, selon la revendication 1 ou 2, caractérisée en ce que le dérivé du benzylidène camphre présente la formule (I) suivante :

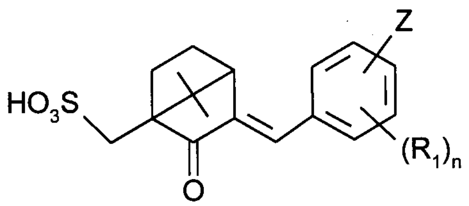

(I)

dans laquelle :

- Z désigne un groupement

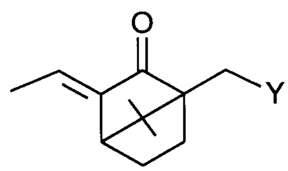

dans lequel Y représente -H ou -$SO_3H$, éventuellement neutralisé,

- n est égal à 0 ou est un nombre allant de 1 à 4($0 \leq n \leq 4$),
- $R_1$, représente un ou plusieurs radicaux alkyle ou alkoxy, identiques ou différents, linéaires ou ramifiés, contenant de 1 à 4 atomes de carbone.

4. Composition selon l'une quelconque des revendications 1 à 3, caractérisée en ce que le dérivé du benzylidène camphre est l'acide benzène 1,4 [di(3-méthylidène campho-10-sulfonique)].

5. Composition selon l'une quelconque des revendications 1 à 4, caractérisée en ce qu'elle se présente sous forme d' une émulsion huile-dans-eau ou sous forme d'une dispersion de sphérules.

6. Composition selon la revendication 5, caractérisée en ce que les sphérules sont des sphérules lipidiques.

7. Composition selon l'une quelconque des revendications précédentes, caractérisée en ce que l'acide kojique représente de 0,05 % à 10 % en poids par rapport au poids total de la composition.

8. Composition selon l'une quelconque des revendications précédentes, caractérisée en ce que ledit filtre ultraviolet représente de 0,1 % à 10 % en poids par rapport au poids total de la composition.

9. Composition selon l'une quelconque des revendications précédentes, caractérisée en ce que'elle contient aussi des adjuvants hydrophiles ou lipophiles.

10. Composition selon l'une quelconque des revendications précédentes, caractérisée en ce que les adjuvants sont

choisis parmi les gélifiants, les conservateurs, les parfums, les charges, les matières colorantes, les actifs autres que l'acide kojique et ledit filtre ultraviolet.

**11.** Utilisation de la composition selon l'une quelconque des revendications précédentes pour traiter les taches cutanées dues au vieillissement.

**12.** Utilisation de la composition selon l'une quelconque des revendications 1 à 10 pour un traitement cosmétique des mains.

**13.** Utilisation de la composition selon l'une quelconque des revendications 1 à 10, pour traiter les rides et/ou les ridules de la peau.

**14.** Utilisation de la composition selon l'une quelconque des revendications 1 à 10 pour tonifier, hydrater et/ou raffermir la peau.

**15.** Utilisation de la composition selon l'une quelconque des revendications 1 à 10 pour préparer une crème destinée au traitement des maladies de peau conférant à cette dernière des taches.

**16.** Procédé non-thérapeutique de traitement cosmétique de la peau, caractérisé en ce qu'il consiste à appliquer sur la peau une composition selon l'une quelconque des revendications 1 à 10.

**Claims**

**1.** Composition for preventing and/or combating skin blemishes and/or for combating ageing of the skin, characterized in that it contains, in a cosmetically and/or dermatologically acceptable medium, kojic acid and an ultraviolet screening agent chosen from the group consisting of benzylidenecamphor and derivatives thereof.

**2.** Composition according to Claim 1, characterized in that the screening agent is a sulpho or sulphonato derivative of benzylidenecamphor.

**3.** Composition according to Claim 1 or 2, characterized in that the benzylidenecamphor derivative has the formula (I) below:

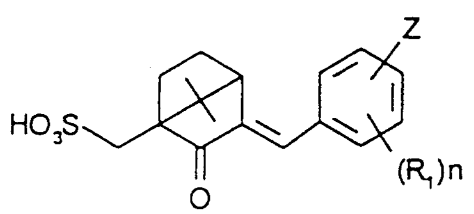

(I)

in which:

- Z denotes a group

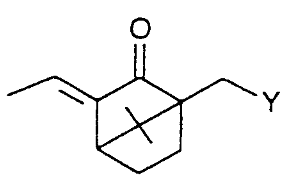

in which Y represents -H or $-SO_3H$, which is optionally neutralized,
- n is equal to 0 or is a number ranging from 1 to 4 ($0 \leq n \leq 4$),
- $R_1$ represents one or more linear or branched alkyl or alkoxy radicals, which may be identical or different, containing from 1 to 4 carbon atoms.

**4.** Composition according to any one of Claims 1 to 3, characterized in that the benzylidenecamphor derivative is benzene-1,4-[di(3-methylidene-10-camphorsulphonic)] acid.

5. Composition according to any one of Claims 1 to 4, characterized in that it is provided in the form of an oil-in-water emulsion or in the form of a dispersion of spherules.

6. Composition according to Claim 5, characterized in that the spherules are lipid spherules.

7. Composition according to any one of the preceding claims, characterized in that the kojic acid represents from 0.05 % to 10 % by weight relative to the total weight of the composition.

8. Composition according to any one of the preceding claims, characterized in that the said ultraviolet screening agent represents from 0.1 % to 10 % by weight relative to the total weight of the composition.

9. Composition according to any one of the preceding claims, characterized in that it also contains hydrophilic or lipophilic adjuvants.

10. Composition according to any one of the preceding claims, characterized in that the adjuvants are chosen from gelling agents, preserving agents, fragrances, fillers, dyes, active agents other than kojic acid and the said ultraviolet screening agent.

11. Use of the composition according to any one of the preceding claims for treating skin blemishes due to ageing.

12. Use of the composition according to any one of Claims 1 to 10 for a cosmetic treatment of the hands.

13. Use of the composition according to any one of Claims 1 to 10 for treating wrinkles and/or fine lines on the skin.

14. Use of the composition according to any one of Claims 1 to 10 to tone, to moisturize and/or to firm the skin.

15. Use of the composition according to any one of Claims 1 to 10 to prepare a cream intended for the treatment of skin diseases which impart blemishes to the skin.

16. Non-therapeutic cosmetic treatment process for the skin, characterized in that it consists in applying to the skin a composition according to any one of Claims 1 to 10.

## Patentansprüche

1. Zusammensetzung zur Verhinderung und/oder Bekämpfung von Hautflecken und/oder der Hautalterung, dadurch gekennzeichnet, daß sie in einem kosmetisch und/oder dermatolisch akzeptablen Medium Kojisäure und ein UV-Filter, das unter Benzylidencampher und dessen Derivaten ausgewählt ist, enthält.

2. Zusammensetzung nach Anspruch 1, dadurch gekennzeichnet, daß das Filter ein Sulfonsäure-Derivat oder ein Sulfonat-Derivat von Benzylidencampher ist.

3. Zusammensetzung nach einem der Ansprüche 1 und 2, dadurch gekennzeichnet, daß das Benzylidencampher-Derivat die folgende Formel (I)

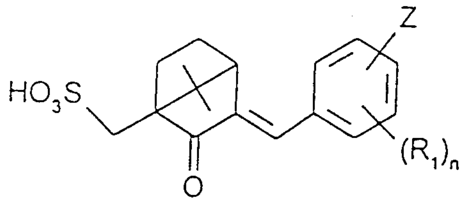

(I)

aufweist, in der bedeuten:

- Z eine Gruppe

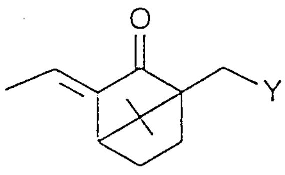

,

worin Y -H oder die Gruppe $-SO_3H$, die gegebenenfalls neutralisiert ist, bedeutet,

- n Null oder eine Zahl von 1 bis 4 ($0 \leq n \leq 4$),
- $R_1$ eine oder mehrere geradkettige oder verzweigte Alkyl- oder Alkoxygruppen mit 1 bis 4 Kohlenstoffatomen, die gleich oder verschieden sind.

4. Zusammensetzung nach einem der Ansprüche 1 bis 3, dadurch gekennzeichnet, daß das Benzylidencampher-Derivat Benzol-1,4-di(3-methylidencampher-10-sulfonsäure) ist.

5. Zusammensetzung nach einem der Ansprüche 1 bis 4, dadurch gekennzeichnet, daß sie in Form einer Öl-in-Wasser-Emulsion oder einer Dispersion von Kügelchen vorliegt.

6. Zusammensetzung nach Anspruch 5, dadurch gekennzeichnet, daß die Kügelchen Lipidkügelchen sind.

7. Zusammensetzung nach einem der vorhergehenden Ansprüche, dadurch gekennzeichnet, daß die Kojisäure 0,05 bis 10 Gew.-%, bezogen auf das Gesamtgewicht der Zusammensetzung, ausmacht.

8. Zusammensetzung nach einem der vorhergehenden Ansprüche, dadurch gekennzeichnet, daß das UV-Filter 0,1 bis 10 Gew.-%, bezogen auf das Gesamtgewicht der Zusammensetzung, ausmacht.

9. Zusammensetzung nach einem der vorhergehenden Ansprüche, dadurch gekennzeichnet, daß sie ferner hydrophile oder lipophile Hilfsstoffe enthält.

10. Zusammensetzung nach einem der vorhergehenden Ansprüche, dadurch gekennzeichnet, daß die Hilfsstoffe unter Gelbildnern, Konservierungsmitteln, Parfums, Füllstoffen, Färbemitteln und Wirkstoffen, die von Kojisäure und dem UV-Filter verschieden sind, ausgewählt sind.

11. Verwendung der Zusammensetzung nach einem der vorhergehenden Ansprüche für die Behandlung von altersbedingten Hautflecken.

12. Verwendung der Zusammensetzung nach einem der Ansprüche 1 bis 10 für die kosmetische Behandlung der Hände.

13. Verwendung der Zusammensetzung nach einem der Ansprüche 1 bis 10 für die Behandlung der Falten und/oder Fältchen der Haut.

14. Verwendung der Zusammensetzung nach einem der Ansprüche 1 bis 10 zur Belebung, zum Schutz und/oder zur Straffung der Haut.

15. Verwendung der Zusammensetzung nach einem der Ansprüche 1 bis 10 zur Herstellung einer Creme, die zur Behandlung von Hautkrankheiten bestimmt ist, die auf der Haut Flecken hervorrufen.

16. Verfahren zur nicht-therapeutischen kosmetischen Behandlung der Haut, dadurch gekennzeichnet, daß es darin besteht, auf die Haut eine Zusammensetzung nach einem der Ansprüche 1 bis 10 aufzutragen.